# EUROPEAN PATENT APPLICATION

(11) **EP 4 125 096 A1**
(43) Date of publication of application: **01.02.2023**
(21) Application number: 21188391.3
(22) Date of filing: 29.07.2021
(51) Int. Cl.: G16H 20/10, G16H 20/30, G16H 20/40, G16H 30/40

(54) **A METHOD AND SYSTEM FOR DETERMINING A TREATMENT PROFILE**

(71) Applicant: Koninklijke Philips N.V., 5656 AG Eindhoven (NL)
(72) Inventor: Damodaran, Mathivanan, 5656 AE Eindhoven (NL); Huijtker, Eefje, 5656 AE Eindhoven (NL); MOESKOPS, Bastiaan Wilhelmus Maria, 5656 AE Eindhoven (NL); WANG, Lu, 5656 AE Eindhoven (NL); Palero, Jonathan Alambra, 5656 AE Eindhoven (NL)
(74) Representative: Philips Intellectual Property & Standards

(57) **Abstract**

According to an aspect, there is provided a computer-implemented method (200) for generating a treatment instruction, the method (200) comprising: receiving (202) first image data relating to the skin of a subject; obtaining (204), based on the first image data, an indication of a location of one or more features of the subject; generating (206), based on the first image data, an initial map of the skin of the subject including the location of the identified features; receiving (208) second image data relating to the skin of the subject during a current treatment session; determining (210), based on the second image data, a treatment parameter relating to a treatment performed in respect of the skin of the subject; generating (212), based on the initial map and the treatment parameter, an updated map of the skin of the subject; and generating (214), based on the updated map, a treatment instruction for delivery to a recipient device.

## Description

### FIELD OF THE INVENTION

The invention relates to determining a treatment profile and, more particularly, to determining a treatment profile based on user behaviour.

### BACKGROUND OF THE INVENTION

Personal care routines may include treatment of blemishes, such as treatment of pimples on the face. Treatment of facial blemishes may involve the application of a topical medication or a medicinal patch, or may involve the use of an electronic device. A person treating facial blemishes would typically treat skin blemishes using their own judgement to distinguish where to perform the treatment, often with the aid of a system or device for visualising blemishes, such as a mirror.

Identifying facial blemishes can be difficult, particularly in cases where, for example, a pimple is not fully developed. Skin blemishes may also be difficult to detect on areas of the body that are not as easily visible, such as the side of the face of a subject or in cases of poor lighting. In contrast, a subject may choose not to treat certain areas of their skin, for example if an area of skin comprises a birth mark. Is can therefore be difficult for a subject to know which areas of their skin to treat and when would be an appropriate time to treat those areas.

It is therefore desirable to improve a treatment regimen of a subject in light of the challenges mentioned above.

### SUMMARY OF THE INVENTION

Subjects with a skin blemish, such as a pimple, dry skin, or the like, may require treatment in order to aid recovery of the skin blemish. In some cases, early detection and treatment of a skin blemish may prevent the skin blemish from worsening. Embodiments disclosed herein provide a mechanism which enables a treatment instruction relating to the skin of a subject to be provided that is personalised to the subject.

According to a first specific aspect, there is provided a computer-implemented method for generating a treatment instruction, the method comprising receiving first image data relating to the skin of a subject; obtaining, based on the first image data, an indication of a location of one or more features of the subject; generating, based on the first image data, an initial map of the skin of the subject including the location of the identified features; receiving second image data relating to the skin of the subject during a current treatment session; determining, based on the second image data, a treatment parameter relating to a treatment performed in respect of the skin of the subject; generating, based on the initial map and the treatment parameter, an updated map of the skin of the subject; and generating, based on the updated map, a treatment instruction for delivery to a recipient device.

The embodiments of the present invention generate, or update, a treatment instruction based on image data of a subject and a treatment parameter, providing for improved skin care. Advantageously, early detection and treatment of skin blemishes, such as pimples or the like, is enabled. Other advantages include that a treatment instruction is tailored to an individual. In this way, areas of the skin that are not treated may be identified and accounted for in the treatment instruction. Similarly, the treatment instruction can be updated to account for an area of the skin that has been treated recently, such that no further treatment is yet required.

In some embodiments, obtaining an indication of a location of one or more features of the subject may comprise one or more of: determining the location of the one or more features of the subject using the first image data; receiving the indication via a user input; and receiving the indication from a treatment device used to treat the one or more features.

The computer-implemented method may, in some embodiments, comprise determining, based on the first image data, the nature of the one or more features.

In some embodiments, the computer-implemented method may comprise generating one or more of: a display instruction for delivery to a display unit, the display instruction configured to cause the display unit to display the updated map, and a communication instruction for delivery to a communication unit, the communication instruction configured to cause the communication unit to communicate the treatment instruction to a recipient.

The computer-implemented method may, in some embodiments, comprise receiving, from a treatment device used to treat the skin of the subject during the current treatment session, treatment data relating to the current treatment session; determining the treatment parameter further based on the received treatment data.

In some embodiments, the computer-implemented method may comprise storing, in a storage device, data for the current treatment session including at least one of: the first image data, the indication of the location of the one or more features, the initial map, the second image data, the determined treatment parameter, the updated map, and the treatment instruction; wherein generating a treatment instruction is further based on stored data relating to a past treatment session.

The recipient device may, in some embodiments, comprise a treatment device used to perform treatment during the current treatment session; and wherein the treatment instruction comprises an instruction to the treatment device to apply or adjust an operating parameter based on the updated map.

In some embodiments, the treatment parameter may comprise one or more of: a location relative to the skin of the subject at which treatment is provided during the current treatment session; an indication of the nature of a treatment provided during the current treatment session; an indication of an operating parameter of a treatment device used during the current treatment session; a date and/or time at which the current treatment session commenced; and a duration of the current treatment session.

The computer-implemented method may, in some embodiments, comprise generating, during the current treatment session, based on the second image data, a revised treatment instruction for delivery to a recipient device.

In some embodiments, each of the one or more features of the subject may comprise a feature selected from a group comprising: a blemish, a pimple, a spot, a wrinkle, a region of scar tissue, a mole, a skin defect, a birthmark, a tattooed region, a bruise and a region of dry skin.

According to a second aspect, the present invention provides a computer program product comprising a non-transitory computer-readable medium, the computer-readable medium having computer-readable code embodied therein, the computer-readable code being configured such that, on execution by a suitable computer or processor, the computer or processor is caused to perform the steps of the methods disclosed herein.

According to a third aspect, the present invention provides a system for generating a treatment instruction comprising an imaging unit configured to acquire image data relating to the skin of a subject; and a processor configured to: receive, from the imaging unit, first image data relating to the skin of a subject; obtain, based on the first image data, an indication of a location of one or more features of the subject; generate, based on the first image data, an initial map of the skin of the subject including the location of the identified features; receive, from the imaging unit, second image data relating to the skin of the subject during a current treatment session; determine, based on the second image data, a treatment parameter relating to a treatment performed in respect of the skin of the subject; generate, based on the initial map and the treatment parameter, an updated map of the skin of the subject; and generate, based on the updated map, a treatment instruction for delivery to a recipient device.

In some embodiments, the system may comprise a treatment device operable to perform treatment of the skin of the subject; wherein the processor is further configured to: transmit the treatment instruction to the treatment device.

The system may, in some embodiments, comprise a presentation device for presenting information to a user; wherein the processor is further configured to: transmit the treatment instruction to the presentation device.

In some embodiments, the system may comprise a user interface configured to receive a user input comprising an annotation associated with the first image data; wherein the processor is configured to generate the treatment instruction further based on the user input.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an example of a subject with facial blemishes;
Fig. 2 is a flowchart of an example of a computer-implemented method for generating a treatment instruction;
Fig. 3 is a flowchart of a further example of a computer-implemented method for generating a treatment instruction;
Fig. 4 is a schematic illustration of a processor in communication with a non-transitory computer readable medium;
Fig. 5 is a schematic illustration of an example of a system for generating a treatment instruction; and
Fig. 6 is a schematic illustration of a further example of a system for generating a treatment instruction.

### DETAILED DESCRIPTION OF THE EMBODIMENTS

It may be desirable for a subject to treat skin blemishes, such as blemishes located on the skin of their body, such as the skin of their face, neck, back, torso, or the like. Challenges associated with treatment of skin blemishes include that, for example, early stage pimples may not be visible to the human eye, certain areas of the skin of the subject may be difficult to examine, and the subject has to gauge how best to treat a skin blemish.

Skin measurement systems may help to overcome these challenges by allowing skin quantification and monitoring of features associated with skin that may detect skin blemishes over time, track user treatment over time and offer information about blemishes that are too small or too faint to be detected by a subject themselves. Advantageously, systems as described herein, which may be referred to as skin measurement systems, may associate measurements of the skin of a subject with how the skin is treated over time, thereby allowing for adaptive, improved and/or personalised treatment recommendations.

The generation of a treatment instruction may provide a treatment instruction for delivery to a recipient device, such as a treatment device, such that the subject is able to treat their skin blemishes in an appropriate way. Advantageously, skin blemishes may be treated objectively rather than the subject treating skin blemishes subjectively.

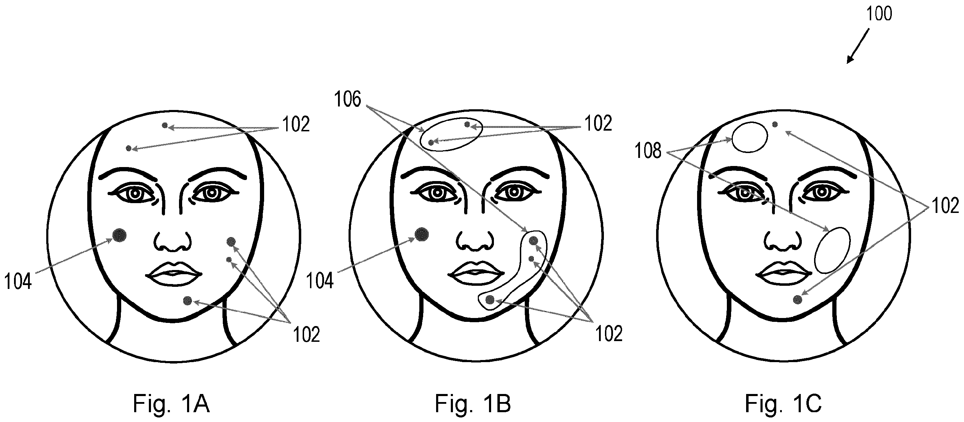

Fig. 1a is an illustration of an example of a subject 100 having features 102, 104 on their skin. A feature of the subject may comprise a feature of the skin of the subject (which may be referred to as "skin features"), including, for example, a blemish, a pimple, a spot, a wrinkle, a region of scar tissue, a mole, a skin defect, a birthmark, a tattooed region, a bruise, a region of dry skin, or the like. Features of the subject may also comprise an eye, a nose, an ear, an eyebrow, a mouth, a shoulder, a neck or a back of the subject, an outline or shape of the face of the subject, or the like. In the illustration shown in Fig. 1a, features 102 comprise pimples whereas feature 104 comprises a birth mark.

In some cases, it may be desirable to treat a skin blemish, such as a pimple, a region of dry skin, or the like, for example to remove the blemish, reduce the size of the blemish, or otherwise change the appearance of the blemish. A skin blemish may be treated via application of a topical, a cream or an ointment, via application of a medicated patch, via application of a "pimple zapper" (e.g. a heated element or radiation applied to the surface of the skin), or the like.

Fig. 1b shows a further illustration of the skin of the subject including features 102, 104, and also including locations 106 of the skin of the subject that have been treated during one or more treatment sessions. Fig. 1b may be obtained by combining the illustration of the subject shown in Fig. 1a with treatment behaviour derived from the one or more treatment sessions. The illustration of the subject shown in Fig. 1b may comprise a heat map, in which areas of the face of the subject that have been treated relatively frequently, or for a relatively long duration, are highlighted differently to those areas that have been treated relatively infrequently or for a relatively short duration. For example, frequently treated areas may appear a first colour (e.g. red or yellow), while less-frequently treated areas may appear a second colour (e.g. blue) or may include no colour, such that the image of the subject is visible.

Fig. 1c shows a further illustration of the skin of the subject 100, which highlights regions of the skin of the subject that are determined to be in need of treatment, as represented by features 102. Notably, feature 104 of Fig. 1b is not present in Fig. 1c, which reflects the nature of the feature (e.g. a birth mark, which requires no treatment). Similarly, areas of the skin of the subject that have already been treated, and which are determined as not requiring any further treatment, may be omitted (as per Fig. 1c); these areas are represented by regions 108.

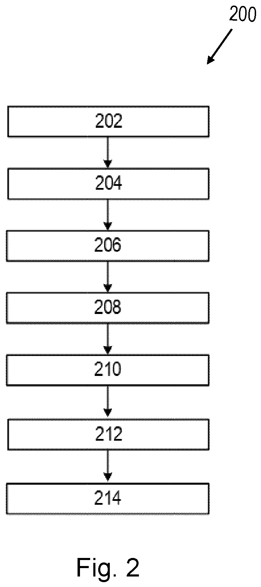

Fig. 2 is a flowchart of an example of a method (e.g. a computer-implemented method) 200 for generating a treatment instruction. The method 200 comprises, at step 202, receiving first image data relating to the skin of a subject. In some examples, first image data may be obtained via an imaging device, such as a camera, a sensor, or the like. The camera may be located in an interactive mirror (e.g. a smart mirror), a smart phone, or the like. In other examples, the first image data may be obtained from a storage medium (e.g. a hard drive), a database, or the like. For example, the first image data may comprise an image of the subject captured using the imaging device, or an image provided (e.g. uploaded) by the subject. In some examples, the storage medium may be located in a smart phone, in the cloud, or the like. The first image data may be obtained prior to a treatment session (e.g. a current treatment session) or during a treatment session.

The method 200 comprises, at step 204, obtaining, based on the first image data, an indication of a location of one or more features of the subject. In some examples, obtaining an indication of a location of one or more features of the subject may comprise one or more of: determining the location of the one or more features of the subject using the first image data; receiving the indication via a user input; and receiving the indication from a treatment device used to treat the one or more features. In some examples, determining the location of the one or more features of the subject using the first image data may comprise applying an algorithm to the first image data in order to identify the one or more features. Other feature detection techniques may additionally or alternatively be used. In examples where the one or more features of the subject may be identified via a user input, a subject may indicate the location of a feature in the first image data (e.g. in an image of their face), for example by touching or circling the one or more features using a touch screen input or using some other input device. In examples in which a treatment device used to treat the one or more features is used to identify the one or more features, a treatment device such as an infrared energy emitter may be applied to a region of the skin of the subject that is associated with a feature, such as a pimple. In such examples, it may be possible to determine the location of the treatment device, or of a treatment element of the device, relative to a reference point, such as a facial landmark of the subject, and this may be used to determine the location of the feature being treated.

In some examples, a location of the one or more features of the subject may be determined with reference to the first image data. For example, a location of a feature may comprise one or more pixel coordinates (e.g. a row and a column) corresponding to the first image data. In some examples, a location of a feature may be determined with reference to one or more body parts of the subject (e.g. an eye, a nose, a shoulder, or the face of the subject), relative to other features, such as facial landmarks (e.g. a corner of an eye, nose or mouth) or the like. The one or more body parts of the subject may be identified from the first image data.

The features of the subject may be identified by comparison of the first image (i.e. an image formed by the first image data), or one or more portions of the first image, to a database of features, by identifying patterns (e.g. changes) in a plurality of image pixels (e.g. in a row or a column of pixels associated with the image data), by identifying a location of a feature relative to at least one other feature, or the like. For example, it may be possible to determine an outline of the face of the subject by identifying a contrast between image pixels associated with the face of the subject compared to image pixels associated with the background behind the subject or compared to image pixels associated with a portion of the subject's skin that does not contain a feature. One or more other features of the face of the subject, such as the eyes, nose, and ears, may be determined with reference to the shape of the face of the subject. These features (e.g. eyes, nose, and ears) of the subject may be expected to be located within defined regions of the face of the subject, which may result in locating these features relatively quickly and accurately. In some examples, a subject may annotate an image (e.g. the first image/first image data) to identify one or more features (e.g. a pimple, a birth mark, an eye, a nose, or the like).

The method 200 comprises, at step 206, generating, based on the first image data, an initial map of the skin of the subject including the location of one or more of the identified features. The initial map of the subject may comprise an outline of a region of skin of the subject (e.g. an outline of the face of the subject). The initial map may be a two-dimensional or three-dimensional representation of the skin of the subject. In some examples, the initial map may represent contours on the skin of the subject, an amount that a pimple protrudes from the skin of the subject, or the like. The initial map of the subject may be generated prior to a treatment session (e.g. a current treatment session) or during a treatment session. The initial map may be displayed to provide treatment guidance. For example, the initial map may highlight regions of skin of the subject that are determined to be in need of treatment.

The method 200 comprises, at step 208, receiving second image data relating to the skin of the subject during a current treatment session. The second image data may be obtained by the same imaging device used to obtain the first image data. Alternatively, the second image data may be obtained by a second imaging device different to the imaging device used to capture the first image data. The first image data and the second image data may each comprise image data associated with the same portion of skin of the subject. In other examples, the second image data may be associated with a different portion of skin of the subject than the first image data. A current treatment session may be considered to be a treatment session in which the subject is applying a treatment to their skin, such as applying an ointment, popping a pimple, or the like. In some examples, a subject may apply treatment to one or more features (e.g. the features of their skin 102). In some examples, the second image data may therefore comprise information relating to treatment of a portion of the skin of the subject. In some examples, two or more images of the skin of the subject may be captured during a current treatment session. The second image data may therefore comprise two or more images captured during a treatment session (e.g. a current treatment session).

In some examples, an orientation of a body part of the subject may be determined from an image (e.g. the first image data and/or the second image data). For example, the orientation of the head of a subject may be determined from the second image.

The method 200 comprises, at step 210, determining, based on the second image data, a treatment parameter relating to a treatment performed in respect of the skin of the subject. In general, the treatment parameter may be considered to represent information about the treatment being performed or applied in respect of the skin of the subject. The treatment parameter may comprise one or more of: a location relative to the skin of the subject at which treatment is provided during the current treatment session; an indication of the nature of a treatment provided during the current treatment session; an indication of an operating parameter of a treatment device used during the current treatment session; a date and/or time at which the current treatment session commenced; and a duration of the current treatment session.

The location of a treatment may be determined with reference to the skin of the subject, with reference to a body part of the subject (e.g. the head of the subject), with reference to the initial map of the skin of the subject, or the like. The nature of the treatment provided during the current treatment session may comprise whether a topical (e.g. a cream, an ointment, or the like) and/or a patch (e.g. a skin patch comprising medicine) was applied to the skin of the subject, whether pimple popping occurred, whether an area of the skin was not treated, the number of times an area of skin was treated, or the like. In some embodiments, the nature of the treatment provided during a treatment session may comprise a treatment applicator type. A treatment applicator type may comprise an indication of the type of device or applicator used to perform the treatment, such as a pimple zapper to heat up the skin at a treatment area, a device to emit light within a certain wavelength range (e.g. laser light, ultraviolet light, infrared light, or the like), a cleansing brush, a finger of the subject, or the like. An indication of an operating parameter of a treatment device used during the current treatment session may comprise an operating temperature of a device (e.g. the temperature of a heated element of a pimple zapper), a wavelength or wavelength range of light used in a light-emitting treatment device, a frequency of light emitted by a light-emitting treatment device (e.g. a device configured to emit bursts of light), a duration of use of a device (e.g. a pimple zapper, a light-treatment device, or the like) (e.g. a duration per facial blemish), or the like.

In some examples, the subject may treat an area of their skin that has not been identified as being or including a feature. In other examples, the subject may not treat an area of their skin that has been identified as a feature or has been identified as an area that contains a feature. A parameter of the treatment device used during a current treatment session may comprise a power setting, a heat setting, a time for which a treatment is applied (e.g. a time for which a heating element is heated), or the like. In some examples, step 210 may be performed during the current treatment session. In other examples, step 210 may be performed after the current treatment session has finished.

In some examples, a treatment parameter may be determined using image-based analytics (e.g. by a processor running an algorithm to process the first image data and/or the second image data). In other examples, a subject may annotate an image (e.g. a first image and/or a second image associated with the first image data and the second image data, respectively) to identify a treatment parameter (e.g. a treatment behaviour or the like). For example, the subject may annotate an image using one or more predefined categories to identify a treatment parameter including, for example, touching, popping, spot treatment, area treatment, massage, or the like. In yet further examples, a treatment parameter may be determined based on data acquired by the treatment device. For example, the treatment device may comprise a pressure sensor, an accelerometer, a magnetometer, or the like. One or more sensors associated with the treatment device may be configured to determine a pressure with which a treatment device is applied to the skin of the subject, an orientation of the treatment device relative to the skin of the subject, a location of treatment (e.g. with reference to the skin of the subject), a time in which a portion of the skin was treated, a time for which the treatment device is used, or the like.

The method 200 comprises, at step 212, generating, based on the initial map and the treatment parameter, an updated map of the skin of the subject. In some examples, the updated map of the skin of the subject may comprise one or more areas of skin of the subject in which a treatment has been applied. The one or more areas of the skin that have had a treatment applied to them may be superimposed with an image (e.g. superimposed with the initial map) of the skin of the subject (e.g. areas of treatment may be overlaid over an image of a subject). In some examples, the updated map may be a heat map or the like. The updated map may highlight areas or regions of skin of the subject that have been treated relatively frequently, for a relatively long duration, or the like. Areas of the skin that have been treated relatively frequency or for a relatively long duration may be highlighted differently to areas of the skin of the subject that have been treated relatively infrequently or for a relatively short duration. For example, an area of the skin of a subject that is treated relatively frequently may be highlighted by a colour (e.g. red), whereas an area of the skin of the subject that is treated relatively infrequently may be highlighted by a different colour (e.g. blue), or by no colour at all.

The method 200 comprises, at step 214, generating, based on the updated map, a treatment instruction for delivery to a recipient device. In some examples, the recipient device may comprise a treatment device used to perform treatment during a treatment session (e.g. the current treatment session). In some examples, the treatment instruction may comprise an instruction to deliver a treatment device control profile to the treatment device. In other examples, the treatment instruction may comprise an instruction to the treatment device to apply or adjust an operating parameter of the treatment device. The instruction to the treatment device to apply or adjust an operating parameter may be based on the updated map. In some examples, a treatment instruction may comprise information relating to a wavelength (or a wavelength range) setting of a treatment device. For example, the treatment device may be configured to emit a specific wavelength of light. In some examples, a treatment instruction may comprise an instruction associated with or defining a duration for which a treatment device is recommended to be used. The duration with which a treatment device is recommended to be used may comprise, for example, a duration per facial blemish, a total duration for which the treatment device is recommended to be used during a current treatment session, or the like. In some examples, a treatment instruction may comprise an instruction to perform treatment in a particular treatment area (e.g. with reference to the initial map and/or the updated map of the skin of the subject). In some examples, the treatment instruction may comprise an instruction to cause a display unit (e.g. a display) to display treatment guidance. For example, the display unit may be instructed to display the updated map, where the updated map may highlight areas of the skin of the subject requiring treatment. The treatment instruction may depend on the type of skin blemish. In some examples, the treatment instruction may be determined using a lookup table, a database, or the like. For example, a particular skin blemish (e.g. a region of dry skin) may be associated with a particular treatment (e.g. application of a moisturising cream), where said association is stored in a database or lookup table. In some examples, the treatment instruction may take account of adjacent skin features (e.g. features of the skin surrounding a region of skin to be treated). For example, if a region of skin to be treated is determined to be adjacent to a birth mark or a tattoo, the treatment instruction may be adjusted such that no damage is caused to the surrounding skin areas (e.g. the treatment instruction may comprise an instruction not to treat that area of the skin, may comprise an instruction to adjust a setting of a treatment device, or the like).

In some examples, a transmit unit may be configured to transmit the treatment instruction to the treatment device. The treatment instruction may be transmitted to the treatment device wirelessly (e.g. Bluetooth (RTM), WiFi or the like) or via a wired connection.

In some examples, an initial map of a subsequent treatment session may comprise an updated map corresponding to a previous treatment session.

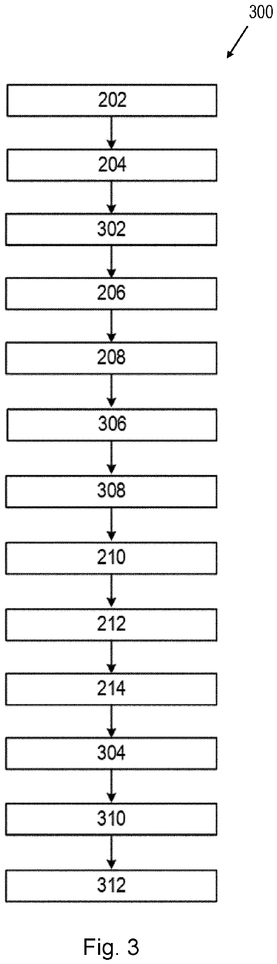

Fig. 3 is a flowchart of a further example of a computer-implemented method 300 for generating a treatment instruction. The method 300 comprises, at step, 302 determining, based on the first image data, the nature of the one or more features. The nature of a feature may comprise a size, colour, shape, or the like, of the feature. The nature of a feature may, in some examples, comprise a type of the feature. Types of features include, for example, skin blemishes associated with the skin of a subject (e.g. a pimple, a region of dry skin, or the like) and features of the body of the subject (e.g. an eye, a nose, a shoulder, or the like).

The method 300 comprises, at step 304, generating one or more of: a display instruction for delivery to a display unit, the display instruction configured to cause the display unit to display the updated map, and a communication instruction for delivery to a communication unit, the communication instruction configured to cause the communication unit to communicate the treatment instruction to a recipient. In some examples, the method 300 may comprise generating a display instruction for delivery to a display unit, the display instruction configured to cause the display unit to display the initial map. For example, the initial map and/or the updated may be displayed and used as treatment guidance. The display unit may be any type of display (e.g. liquid-crystal display or the like). The communication unit may be a speaker or the like. The communication unit may allow treatment instructions to be provided to a subject aurally. The display unit and/or communication unit may be located in a smart mirror, in a treatment device, in a smart phone, or the like. In some embodiments, the term "presentation unit" may be considered to cover both display units and communication units. A presentation unit may be configured to present the treatment instruction to a recipient.

The method 300 comprises, at step 306, receiving, from a treatment device used to treat the skin of the subject during the current treatment session, treatment data relating to the current treatment session. Treatment data relating to a treatment session may comprise a duration for which a treatment device was used, a location of an area of the skin of the subject that was treated, an orientation of a treatment device relative to the skin of the subject, an applicator type (e.g. a device type, a subject's finger, or the like), a wavelength of light used by a light-treatment device, a temperature of a heating element used in a heat-treatment device (e.g. a pimple zapper), or the like.

The method 300 comprises, at step 308, determining the treatment parameter further based on the received treatment data. The treatment parameter may be updated based on the received treatment data. For example, if it is determined that an area of the skin of the subject has been treated (e.g. by application of a cream comprising medicine to an area of the skin of the subject), then the treatment parameter may be updated such that it comprises information to this effect. In some examples, an updated map be generated, or updated, based on the received treatment data and/or an initial map of the skin of the subject. In some examples, a treatment instruction may be generated, or updated, based on the updated map such that the treatment instruction does not include a recommendation to (or includes a recommendation not to) treat said area of skin during a subsequent treatment session. In some examples, if it is determined that a pimple zapper was applied to an area of skin of the subject (e.g. determined using the second image data and/or the received treatment data), then the treatment instruction may be generated, or updated, such that it does not include an instruction to treat said area of skin using a pimple zapper during a subsequent treatment session. In some examples, if it is determined that a pimple zapper was applied to an area of skin of the subject for a duration below a threshold amount, then the treatment instruction may be generated, or updated, such that it includes an instruction to treat said area of skin during a subsequent treatment session. In some examples, the treatment parameter may be based on the second image data and/or the received treatment data.

The method 300 comprises, at step 310, storing, in a storage device (e.g. a memory), data for the current treatment session including at least one of: the first image data, the indication of the location of the one or more features, the initial map, the second image data, the determined treatment parameter, the updated map, and the treatment instruction. A storage device may be located in a smart mirror, a smart phone, a computer, the cloud, or the like.

Generating a treatment instruction may be further based on stored data relating to a past treatment session. In some examples, a treatment instruction may be generated based on one or more past treatment sessions and/or a current treatment session. Data (e.g. stored data) relating to a past treatment session may comprise one or more of: the first image data, the indication of the location of the one or more features, the initial map, the second image data, the determined treatment parameter, the updated map, and the treatment instruction. For example, if a subject does not treat an area of skin that is identified as comprising a feature (e.g. a birth mark) during one or more past treatment sessions and/or a current treatment session, then the generated treatment instruction may be updated such that it does not contain an instruction to treat the untreated area of skin. As another example, if it is determined that an area of skin has been treated during a past treatment session, the generated treatment instruction may be updated such that it does not contain an instruction to treat the same area of skin again during a subsequent treatment session. If a medicine (e.g. a cream) is to be applied once every two days, and it is determined from a past treatment session (e.g. a previous treatment session immediately before the current treatment session) that the medicine was not applied to the skin of the subject during the past treatment session, then the generated treatment instruction may comprise an instruction to treat the skin of the subject using said medicine (e.g. during the treatment session immediately following the current treatment session). It may be determined, based on an analysis of the stored data relating to a past treatment session, that no region of the skin was treated. Alternatively, it may be determined that one or more portions of the skin were treated and/or that one or more portions of the skin were not treated. The one or more portions of the skin that were treated, and/or the one or more portions of the skin that were not treated, may correspond to one or more different features of the skin, respectively. The generated treatment instruction may therefore comprise an instruction to treat the entire portion of skin, or, alternatively, an instruction to treat one or more portions of the skin that were, or were not, treated during a past treatment session.

The method 300 comprises, at step 312, generating, during the current treatment session, based on the second image data, a revised treatment instruction for delivery to a recipient device. In some examples, one or more revised treatment instructions may be generated during a current treatment session. In some examples, the subject may be applying a treatment to their skin using a recipient device (e.g. a treatment device), wherein the recipient device is configured to operate based on a treatment instruction from a previous treatment session. In some examples, the recipient device may comprise a display (e.g. a smart mirror, a smart phone, or the like) configured to display a treatment instruction. In this case, the treatment instruction may comprise an instruction to display the updated map of the skin of the subject, the updated map highlighting one or more areas of the skin of the subject that require treatment. During the current treatment session, it may be determined that an area of the skin of the subject has been treated (e.g. based on the second image data and/or treatment data relating to a treatment session). A revised treatment instruction may therefore be generated based on the second image data and/or the treatment data relating to a treatment session. The revised treatment instruction may be delivered to the recipient device. The recipient device may update an operating parameter of a treatment device, update a display comprising guidance based on the treatment instruction, or the like, during a current treatment session and before the current treatment session has ended.

In some examples, a processor associated with a smart phone, a smart mirror, or the like, may be used to perform one or more of the steps of the methods disclosed herein (e.g. methods 200, 300). In other examples, an image of a portion of the skin of a subject may be transmitted to a processor configured to perform one or more of the steps of the methods disclosed herein (e.g. methods 200, 300). Transmittal of image data between a smart phone or the like and a processor configured to perform one or more of the steps of the methods disclosed herein may be initiated via a user interface, via an application on a smart phone, or the like.

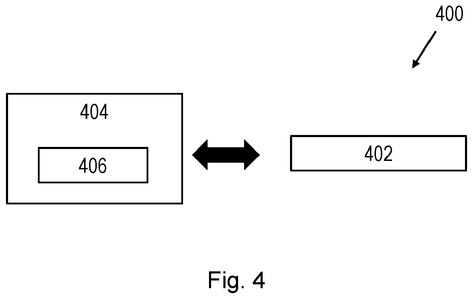

An aspect of the invention relates to a computer program product. Fig. 4 is a schematic illustration of a non-transitory computer-readable medium 404 in communication with a processor 402. The computer-readable medium 404 has computer-readable code 406 embodied therein, the computer-readable code being configured such that, on execution by a suitable computer or processor 402, the computer or processor is caused to perform steps of a method, such as the method 200 and/or the method 300, as described above. The processor 402 may form part of, or be accessible by a computing device, such as a desktop computer, a laptop computer, or the like, or may comprise a server, accessible via a cloud-computing environment.

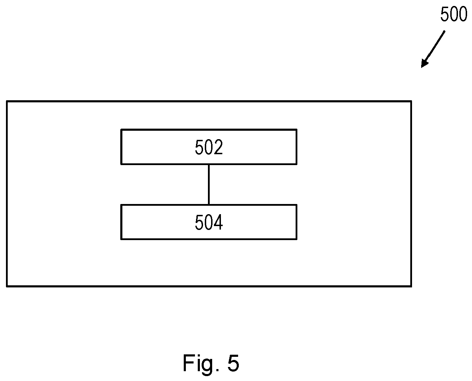

Fig. 5 is a schematic illustration of an example of a system 500 for generating a treatment instruction. The system 500 comprises an imaging unit 504 configured to acquire image data relating to the skin of a subject, and a processor 502. The processor 502 is in communication with the imaging unit 504. In some embodiments, the processor 502 may be configured to operate or control the imaging unit 504 and/or one or more other components. The processor 502 is configured to receive, from the imaging unit, first image data relating to the skin of a subject. The processor 502 is further configured to obtain, based on the first image data, an indication of a location of one or more features of the subject. The processor 502 is further configured to generate, based on the first image data, an initial map of the skin of the subject including the location of the identified features. The processor 502 is further configured to receive, from the imaging unit, second image data relating to the skin of the subject during a current treatment session. The processor 502 is further configured to determine, based on the second image data, a treatment parameter relating to a treatment performed in respect of the skin of the subject. The processor 502 is further configured to generate, based on the initial map and the treatment parameter, an updated map of the skin of the subject. The processor 502 is further configured to generate, based on the updated map, a treatment instruction for delivery to a recipient device.

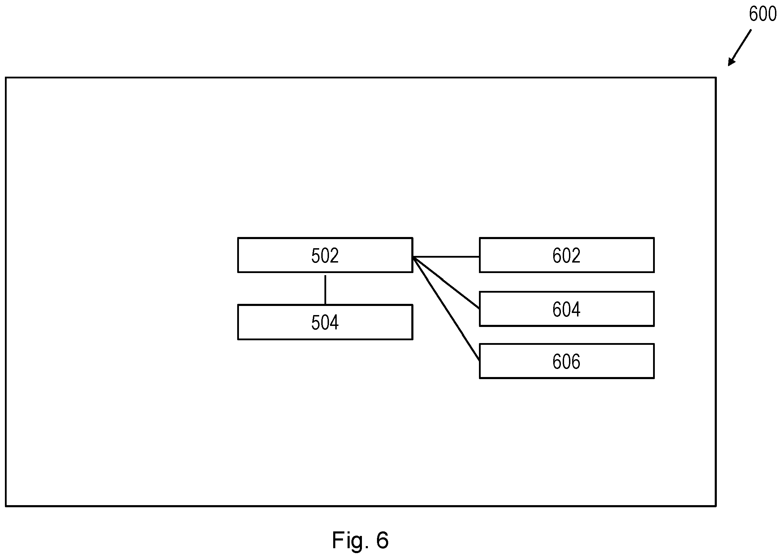

Fig. 6 is a schematic illustration of a further example of a system 600 for generating a treatment instruction. The system 600 may comprise an imaging unit 504 configured to acquire image data relating to the skin of a subject, and may comprise a processor 502. The processor 502 may be in communication with the imaging unit 504. In some embodiments, the processor 502 may be configured to operate or control the imaging unit 504 and/or one or more other components.

In some embodiments, the system 600 may comprise a treatment device 602 operable to perform treatment of the skin of the subject. The processor 502 may be further configured to transmit the treatment instruction to the treatment device 602.

In some embodiments, the system 600 may comprise a presentation device 604 for presenting information to a user. The processor 502 may be further configured to transmit the treatment instruction to the presentation device 604. The presentation device may comprise, or be similar to, the presentation unit, display unit or communication unit discussed above.

In some embodiments, the system 600 may comprise a user interface 606 configured to receive a user input comprising an annotation associated with the first image data and/or the second image data. The processor 502 may be configured to generate the treatment instruction further based on the user input.

The system 500 and/or system 600 may be configured to perform any of the method steps disclosed herein.

The processor 402, 502 can comprise one or more processors, processing units, multicore processors or modules that are configured or programmed to control the system 500, 600 in the manner described herein. In particular implementations, the processor 402, 502 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for generating a treatment instruction, the method comprising:
receiving first image data relating to the skin of a subject;
obtaining, based on the first image data, an indication of a location of one or more features of the subject;
generating, based on the first image data, an initial map of the skin of the subject including the location of the identified features;
receiving second image data relating to the skin of the subject during a current treatment session;
determining, based on the second image data, a treatment parameter relating to a treatment performed in respect of the skin of the subject;
generating, based on the initial map and the treatment parameter, an updated map of the skin of the subject; and
generating, based on the updated map, a treatment instruction for delivery to a recipient device.

2. A method according to claim 1, wherein obtaining an indication of a location of one or more features of the subject comprises one or more of: determining the location of the one or more features of the subject using the first image data; receiving the indication via a user input; and receiving the indication from a treatment device used to treat the one or more features.

3. A method according to claim 1 or claim 2, further comprising:
determining, based on the first image data, the nature of the one or more features.

4. A method according to any of the preceding claims, further comprising generating one or more of:
a display instruction for delivery to a display unit, the display instruction configured to cause the display unit to display the updated map, and
a communication instruction for delivery to a communication unit, the communication instruction configured to cause the communication unit to communicate the treatment instruction to a recipient.

5. A method according to any of the preceding claims, further comprising:
receiving, from a treatment device used to treat the skin of the subject during the current treatment session, treatment data relating to the current treatment session;
determining the treatment parameter further based on the received treatment data.

6. A method according to any of the preceding claims, further comprising:
storing, in a storage device, data for the current treatment session including at least one of: the first image data, the indication of the location of the one or more features, the initial map, the second image data, the determined treatment parameter, the updated map, and the treatment instruction;
wherein generating a treatment instruction is further based on stored data relating to a past treatment session.

7. A method according to any of the preceding claims, wherein the recipient device comprises a treatment device used to perform treatment during the current treatment session; and
wherein the treatment instruction comprises an instruction to the treatment device to apply or adjust an operating parameter based on the updated map.

8. A method according to any of the preceding claims, wherein the treatment parameter comprises one or more of: a location relative to the skin of the subject at which treatment is provided during the current treatment session; an indication of the nature of a treatment provided during the current treatment session; an indication of an operating parameter of a treatment device used during the current treatment session; a date and/or time at which the current treatment session commenced; and a duration of the current treatment session.

9. A method according to any of the preceding claims, further comprising:
generating, during the current treatment session, based on the second image data, a revised treatment instruction for delivery to a recipient device.

10. A method according to any of the preceding claims, wherein each of the one or more features of the subject comprises a feature selected from a group comprising: a blemish, a pimple, a spot, a wrinkle, a region of scar tissue, a mole, a skin defect, a birthmark, a tattooed region, a bruise and a region of dry skin.

11. A computer program product comprising a non-transitory computer-readable medium, the computer-readable medium having computer-readable code embodied therein, the computer-readable code being configured such that, on execution by a suitable computer or processor, the computer or processor is caused to perform the method of any of the preceding claims.

12. A system for generating a treatment instruction, the system comprising:
an imaging unit configured to acquire image data relating to the skin of a subject; and
a processor configured to:
receive, from the imaging unit, first image data relating to the skin of a subject;
obtain, based on the first image data, an indication of a location of one or more features of the subject;
generate, based on the first image data, an initial map of the skin of the subject including the location of the identified features;
receive, from the imaging unit, second image data relating to the skin of the subject during a current treatment session;
determine, based on the second image data, a treatment parameter relating to a treatment performed in respect of the skin of the subject;
generate, based on the initial map and the treatment parameter, an updated map of the skin of the subject; and
generate, based on the updated map, a treatment instruction for delivery to a recipient device.

13. A system according to claim 12, further comprising:
a treatment device operable to perform treatment of the skin of the subject;
wherein the processor is further configured to:
transmit the treatment instruction to the treatment device.

14. A system according to claim 12 or claim 13, further comprising:
a presentation device for presenting information to a user;
wherein the processor is further configured to:
transmit the treatment instruction to the presentation device.

15. A system according to any of claims 13 to 14, further comprising:
a user interface configured to receive a user input comprising an annotation associated with the first image data;
wherein the processor is configured to generate the treatment instruction further based on the user input.
